# EUROPEAN PATENT APPLICATION

(11) **EP 1 745 819 A2**
(43) Date of publication of application: **24.01.2007**
(21) Application number: 06116263.2
(22) Date of filing: 29.06.2006
(51) Int. Cl.: A61N 5/06, A61N 5/067

(54) **Instrument for treating amblyopia using automatic frequency conversion laser**

(30) Priority: 19.07.2005 CN 200520114140; 16.08.2005 CN 200520103791; 18.08.2005 WO PCT/CN2005/001285
(71) Applicant: Fung, Danlai, Wanchai, Hong Kong (CN)
(72) Inventor: Fung, Danlai, 302-308 Hennessy Road, Wanchai Hong Kong (CN); Li, Zhisheng c/o Beijing Radiant Children's, Beijing 100036 (CN)
(74) Representative: Intes, Didier Gérard André

(57) **Abstract**

The present invention relates to an instrument for treating amblyopia using automatic frequency conversion laser, comprises a laser generator, a circular lightproof system installed on the output light path and a control circuit, also comprises an angular rotation expander (2) for adjusting height and light path output angle. The angular rotation expander (2) comprises a primary drawtube (203) having a primary lens (2031), set outside of a secondary drawtube (201) having a secondary lens (2011). The primary drawtube (203) is set outside of the secondary lens (2011) and can reciprocate along it. A retainer (202) is equipped on the upper position of the secondary drawtube (201). There is an opening (204) on one side of the primary drawtube (201) for leaving the light out, a reflector (205) is set inside of the primary lens (2031) facing the opening (204). Light outputted by the laser generator is transmitted directly to the secondary lens (2011), and then to the primary lens (2031), and finally is sent out from the opening (204) after reflected by the reflector (205).

## Description

### FIELD OF THE INVENTION

The present invention relates to a medical instrument, especially relates to an instrument for treating amblyopia using automatic frequency conversion laser.

### BACKGROUND OF THE INVENTION

Amblyopia is very common in children, and it belongs to one kind of non-organic pathological change in eye itself. When vision after being corrected is still lower than 0.9 (normal), it is related with eyeground retina centrum cell function, optic nerve conduction function and visual center function.

Recent study indicates that amblyopia and pseudomyopia are well treated when eyes are irradiated by a beam whose wave length is close to the visual cell sensitive wave length and retina nerve cell is stimulated benignly and circularly by frequency conversion light wave which has very strong visual response sensitivity. In 1994, the present applicant designed a first generation instrument for treating amblyopia using automatic frequency conversion laser, with CN patent number 9411755.6, which comprises a helium neon laser generator, a direct current precision buncher and a main control circuit displaced in a cabinet. It is an instrument that implements frequency conversion laser treatment on pathological change spot of an eye by using the automatic frequency conversion laser technology. Since it is manufactured, it is very beneficial to patients with amblyopia. But it also has some defects, 1. the helium neon laser generator outputs light from a certain angle and height, so when facing different patients with different height, the entire instrument must be moved in order to adjust its height and angle, or let the patients bend, turn, etc., so that the laser beam sent out by the helium neon laser generator can aim at the patient's eye for laser irradiation. This brings much inconvenience to the operator and the patient. 2. working status of frequency conversion laser can't be displayed and intensity of frequency conversion laser can't be adjusted. 3. There is no timing device, so the operator must control time himself, and it takes human resources.

### SUMMARY OF THE INVENTION

An object of the present invention is to provide an instrument for treating amblyopia using automatic frequency conversion laser, which is conveniently adjusted and operated and has good use effect.

To achieve the object, the present invention adopts following solutions: an instrument for treating amblyopia using automatic frequency conversion laser, comprises a laser generator, a circular lightproof system installed on the output light path and a control circuit, also comprises an angular rotation expander for adjusting height and light path output angle, said angular rotation expander comprises a primary drawtube having a primary lens, set outside of a secondary drawtube having a secondary lens. The primary drawtube is set outside of the secondary lens and can reciprocate along it. There is an opening on one side of the primary drawtube for leaving the light out, a reflector is set inside of the primary lens facing the opening. Light outputted by the helium neon laser generator is transmitted directly to the secondary lens, and then to the primary lens, and finally is sent out from the opening after reflected by the reflector.

Said secondary drawtube is connected with the output light path of the helium neon laser generator by a light-adjusting unit.

In order to achieve a better treatment, wave length of the light outputted by the helium neon laser generator is from 630.0 to 650.0nm.

Said laser generator is a helium neon laser generator, or a semiconductor laser generator.

The control circuit comprises a loop timing generating circuit, a controlled shunt constant voltage source, a steady speed drive circuit, a circuit for measuring speed, an acousto-optics indicating circuit and a power circuit; the low-voltage power source of the power circuit provides a work power source for the loop timing generating circuit and the acousto-optics indicating circuit, and the high-voltage power circuit provides a work power source for the helium neon laser generator; the loop timing generating circuit sends out timing signals and provides a stable voltage for the direct current buncher through the steady speed drive circuit.

The invention has following advantages: it has good results in treating amblyopia; the cure rate is high; there is no side-effect; compared with the prior art, the period of treatment may be reduced to 1/10, and it is convenient to be used.

### BRIEF DESCRIPTION OF THE DRAWINGS

FIG. 1 is a schematic diagram illustrating the structure of the invention.
FIG. 2 is a schematic diagram illustrating the structure of the angular rotation expander.
FIG. 2-A shows that part of FIG. 2 designated by the circle A to a larger scale.
FIG. 3 is a schematic diagram illustrating the structure of the light-adjusting unit.
FIG. 4 is a functional block diagram of the control circuit of the invention.
FIG. 5 is an electronic schematic diagram of the control circuit of the invention.

### DETAILED DESCRIPTION OF THE INVENTION

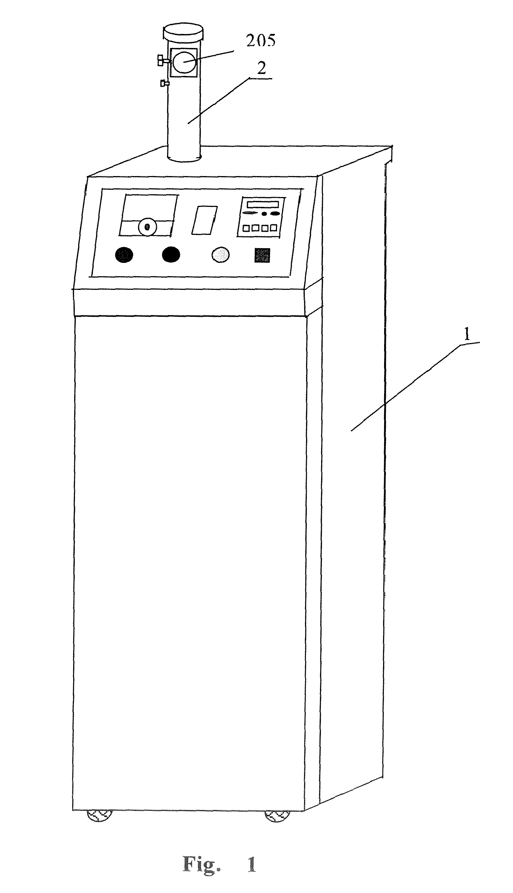

As shown in FIG. 1, the present invention comprises a laser generator (the present embodiment adopts a helium neon laser generator), a circular lightproof system (conventional, details will not be given here, which is installed in the cabinet 1) installed on the output light path, and a control circuit. The present invention features that an angular rotation expander 2 is installed to make the height and angle of the light outputted by the laser generator be adjustable. There are provided on the front panel of the cabinet a lamp for indicating the status of stroboflash, a time switch and a display instrument, so that it is very convenient for the operator to operate and control it.

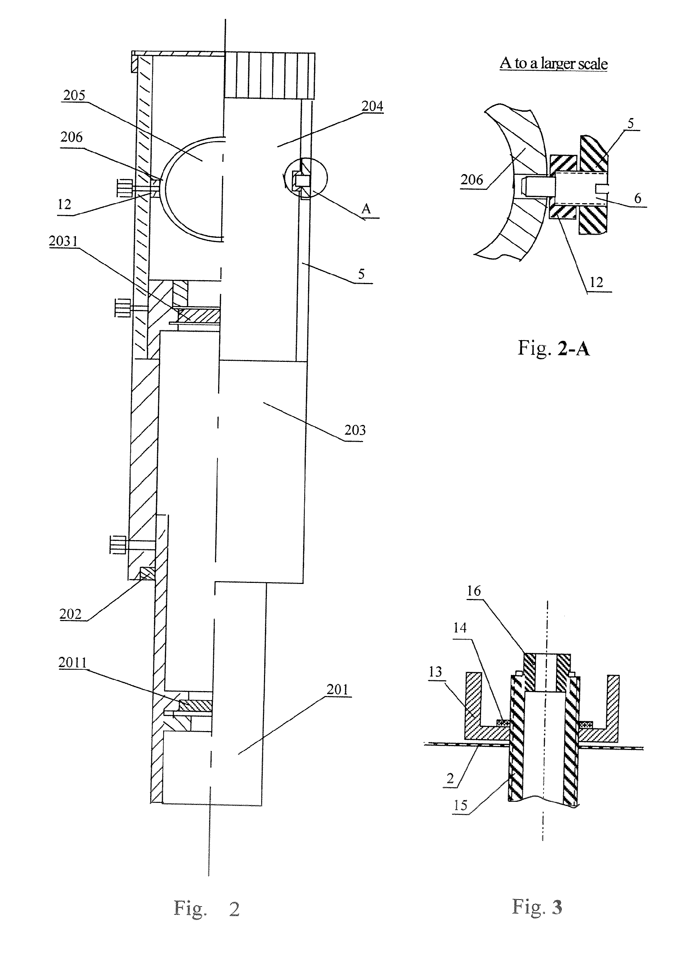

As shown in FIG. 2 and FIG. 2-A, the angular rotation expander 2 especially designed in the present invention comprises: a primary drawtube 203 having a primary lens 2031, and a secondary drawtube 201 having a secondary lens 2011. The primary drawtube is set outside of the secondary drawtube and can reciprocate along it. A retainer 202 is equipped on the upper position of the secondary drawtube. There is an opening 204 on one side of the primary drawtube for leaving the light out, and a reflector 205 is set inside of the primary drawtube facing the opening. The reflector supporter 206 is fixed on a tumbler 5 by a bolt 6 and a washer nut 12.

The secondary drawtube of the angular rotation expander is connected with the output light path of the laser generator by a light-adjusting unit (with reference to FIG. 3), which comprises a light adjustment rack 15, outside of which the secondary drawtube of the angular rotation expander is set fastened by a splicing sleeve 13 and a clamp nut 14, and in the center of which is an optical fiber splice.

After research, visual cell is the most sensitive to the wave length 630.0 to 650.0 nm, and is the most easily activated so as to make its function recover and achieve a goal of treating amblyopia, so the output light wave length of the helium neon laser generator is from 630.0 to 650.0nm.

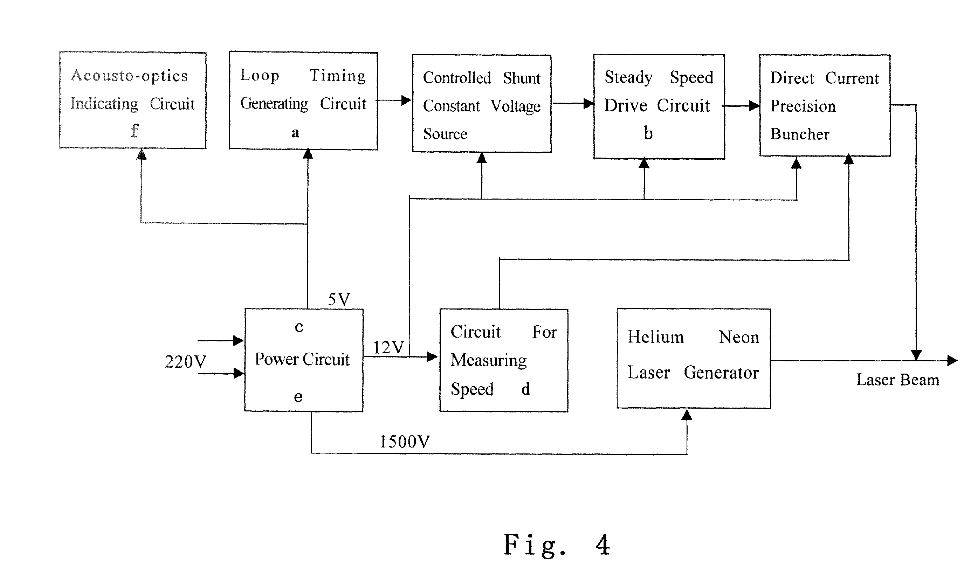

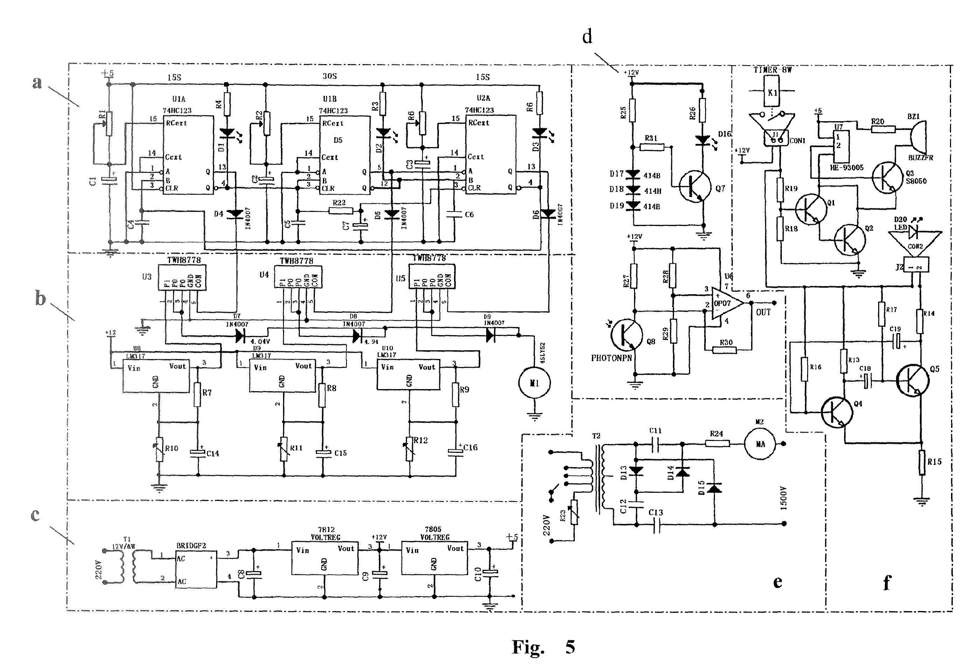

FIG. 4 and FIG. 5 are electronic schematic diagrams of the invention. Said circuit comprises a loop timing generating circuit, a controlled shunt constant voltage source, a steady speed drive circuit, a circuit for measuring speed, acousto-optics indicating circuit and a power circuit.

As shown in dotted line frame a in FIG. 5, the loop timing circuit for generating 15 seconds, 30 seconds and 15 seconds circular timings comprises three monostable multivibrators U1A, U1B, U2A whose type is 74123, wherein: R1, R2, R3, C1, C2, C3 are timing devices of the timing circuit. The negative output end Q⁻ of U1A connects clear end and input end B of U1B, and also connects clear end of U2A through integral delay circuit composed by R22 and C7, negative output end Q⁻ of U1B is connected with input end B of U2A, negative output end Q⁻ of U2A is connected with input end B of U1A, red, yellow, green LED D1, D2, D3 are fixed between +5V power end and each negative output end Q⁻ of U1A, U1B, U2A, each output end Q of U1A, U1B, U2A sends out timing signals through diode D4, D5, D6.

The feature of said circuit is that U1A is always in operation first after being powered up, U1B, U2A can only be in operation after triggered by U1A in order. It is assured by the locking pattern that only one timing can be outputted at one moment. Work progress is: U1A is in operation after being powered up, Q end of U1A outputs 5V high level timing signal, simultaneously U1B, U2A is in 0 state under the control of Q⁻ of U1A, and the red LED D1 is lighted up. U1A finishes work after 15 seconds, U1B is triggered to work by the signal of Q end of U1A descending from high level to low level, Q end of U1B outputs 5V high level timing signal, and the yellow LED D2 is lighted up. When U1B is triggered by Q end of U1A, due to delay of R22 and C7, said trigger signal doesn't take effect on U2A, when U1B is in operation, its Q end locks U2A in 0 state automatically, After 30 seconds U1B finishes work, and triggers U2A to work, Q end of U2A outputs high level timing signal, and the green LED D3 is lighted up. After 15 seconds U2A finishes work, its ascending signal of Q⁻ end triggers U1A in operation once again, so that a circulation, whose period is one minute, is formed.

As shown in dotted line frame b in FIG. 5, power switches U3, U4, U5, which correspond to the three timings of 15 seconds, 30 seconds, 15 seconds, constitute the steady speed drive circuit. Constant voltage chips U8, U9, U10, which provide three constant voltages, and three isolation diodes D7, D8, D9 for isolating power switch output constitute the controlled shunt constant voltage source. Wherein, the three timing signals of the loop timing circuit are connected with the control end of power switches U3, U4, U5 via D4, D5, D6, the output ends of constant voltage chips U8, U9, U10 are connected with the direct current buncher M1 through respective power switch U3, U4, U5 and isolation diodes D7, D8, D9. Three precise voltages of 4.04V, 4.94V, 6.02V generated respectively by U8, U9, U10 are periodically used as working power source of the direct current buncher M1 due to the effect of the timing signals. The output voltage of the circuit may be flexibly adjusted according to characteristics of the buncher's voltage-frequence. R10 to R12 are regulating resistances of respective constant voltage chip.

The three power switches U3, U4, U5 correspond to the three timings of 15 seconds, 30 seconds, 15 seconds. When the first timing (Q of U1A is in effect) is in operation, U3 works, and adds the first voltage 4.04V generated by U8 on the power input end of the buncher M through diode D7, so as to make the buncher generate a rotating speed of 7.5 HZ. When U3 works, the control timings of U4, U5 are not in effect, so U4, U5 do not work. D4, D5, D6's effect is to isolate the timing circuit from the drive circuit. D7, D8, D9's effect is to prevent the buncher from being affected by the drive circuit.

The circuit for measuring speed adopted in the present invention is disclosed in dotted line frame d. It adopts photoelectricity conversion technology, and comprises transistors Q7 and Q8, operational amplifier U6 (OP07) and its necessary accessories. The transistor Q7 drives the infrared luminotron D16 to shine, light sent out by D16 can be reflected to the light-sensitive receiving tube Q8 only when it is blocked by something, the rotor blade of the buncher is placed on the light path of D16, the buncher's turning around will lead to that the blades block the light at intervals, such that the collector of Q8 will receive turning signals of the buncher. After being amplified by U6, the turning signals become square-wave signals whose amplitude is 12V, the rotating speed of the buncher can be determined by adding the square-wave signals on a frequency meter.

A low voltage power circuit is disclosed in dotted line frame c. After 220V AC is transformed by T1, rectified by B1, filtered by C8, stabilized by constant voltage chips 7812 and 7805, two kinds of power sources of direct current 12V and 5V are formed.

A high voltage power circuit is disclosed in dotted line frame e. After being transformed by transformer T2, 220V AC becomes 500V AC, which is then triplevoltage-rectified by D13 to D15 and is then filtered, so as to form a direct current working voltage 1500V as working power of the helium neon laser generator, a current meter is connected to the circuit, the gauge outfit is set on the panel of the cabinet, laser intensity of the helium neon laser generator is set by an adjusting switch (i.e. adjusting R23).

The acousto-optics indicating circuit, which is controlled by a timing switch 6, is disclosed in dotted line frame f. Details will not be given here.

Operational principle of the invention shall be described by referring to FIG. 4:

The power circuit provides direct current working voltage of 5V and 1500V to the loop timing generating circuit and the helium neon laser generator, and provides direct current working voltage of 12V to the controlled shunt constant voltage source, the steady speed drive circuit, the direct current precision buncher and the circuit for measuring speed. The loop timing generator outputs three circular timings of 15 seconds, 30 seconds, 15 seconds to the input end of the controlled shunt constant voltage source, the steady speed drive circuit is connected between the controlled shunt constant voltage source and the direct current precision buncher, so as to make the direct current precision buncher obtain three constant voltages needed by circular rotating speed 7.5HZ±1, 10HZ±1, 12.5HZ±1. The output end of the circuit for measuring speed is connected with the input end of the direct current precision buncher. Said circuit can measure the rotating speed of the direct current precision buncher, so as to assure the rotating speed of the buncher be exactly 7.5HZ±1, 10HZ±1, 12.5HZ±1. When the buncher is in operation under said rotating speed, the double frequency lightproof panel in the rotor axis forms a lightproof system whose frequency is 15HZ±2, 20HZ±2, 25HZ±2, the permanent laser beam sent out by the helium neon laser generator is adjusted to a circularly changeable beam of the frequency, and then the height and angle of the output light path is further adjusted by the angular rotation expander so as to make the light sent out aim at the patient's eye, stimulation is produced, sense conduction of visual information and processing and analyzing ability of visual center are increased. Retina microcirculation is improved, and metabolism is promoted.

When in use, turn on the power switch, frequency modulation switch, laser intensity adjusting switch in turn, adjust the gauge outfit at about 6mA, make the laser beam sent out by the instrument for treating amblyopia using automatic frequency conversion laser aim at the patient's eye for laser irradiation , so as to conduct a laser stimulus.

## Claims

1. An instrument for treating amblyopia using automatic frequency conversion laser, **characterized in that** it comprises a laser generator, a circular lightproof system installed on the output light path and a control circuit, as well as an angular rotation expander (2) for adjusting height and angle of an output light path.

2. The instrument for treating amblyopia using automatic frequency conversion laser of claim 1, wherein the angular rotation expander (2) comprises a primary drawtube (203), a secondary drawtube (201), a retainer (202), an opening (204), and a reflector (205), the primary drawtube (203) having a primary lens (2031) setting outside of and reciprocating along the secondary drawtube (201) which has a secondary lens (2011), the retainer (202) being located on the upper position of the secondary drawtube (201), the opening (204) in one side of the primary drawtube (203) for the light going through, the reflector (205) setting inside of the primary lens (2031) and facing the opening (204), so that light path of the laser generator goes through the secondary lens (2011) and the primary lens (2031), and is then reflected out of the opening (204) by the reflector (205).

3. The instrument for treating amblyopia using automatic frequency conversion laser of claim 1, wherein the secondary drawtube is connected with the output light path of the laser generator by a light-adjusting unit.

4. The instrument for treating amblyopia using automatic frequency conversion laser of claim 1, wherein wave length of the light outputted by the laser generator is from 630.0 to 650.0nm.

5. The instrument for treating amblyopia using automatic frequency conversion laser of claim 1, wherein the laser generator is a helium neon laser generator.

6. The instrument for treating amblyopia using automatic frequency conversion laser of claim 1, wherein the laser generator is a semiconductor laser generator

7. The instrument for treating amblyopia using automatic frequency conversion laser of claim 5, wherein the control circuit comprises a loop timing generating circuit, a controlled shunt constant voltage source, a steady speed drive circuit, a circuit for measuring speed, an acousto-optics indicating circuit and a power circuit; the power circuit having a low-voltage power source providing a work power source for the loop timing generating circuit and the acousto-optics indicating circuit, and a high-voltage power circuit providing a work power source for the helium neon laser generator; the loop timing generating circuit sending out timing signals and providing a stable voltage for the direct current buncher through the steady speed drive circuit.
